# EUROPÄISCHE PATENTSCHRIFT

(11) **EP 1 605 829 B1**
(45) Veröffentlichungstag und Bekanntmachung des Hinweises auf die Patenterteilung: **14.06.2017**
(21) Anmeldenummer: 04722853.1
(22) Anmeldetag: 24.03.2004
(51) Int. Cl.: A61B 6/14

(54) **INTRAORALER RÖNTGENSENSOR**
INTRAORAL X-RAY SENSOR
DÉTECTEUR DE RAYONS X INTRAORAL

(30) Priorität: 24.03.2003 DE 10313044; 06.10.2003 DE 10346287
(43) Veröffentlichungstag der Anmeldung: 21.12.2005
(73) Patentinhaber: Kaltenbach & Voigt GmbH, 88400 Biberach (DE)
(72) Erfinder: HACK, Alexander, 88400 Biberach (DE)
(74) Vertreter: Thun, Clemens
(86) Internationale Anmeldenummer: PCT/EP2004/003126
(87) Internationale Veröffentlichungsnummer: WO 2004/084730

(56) Entgegenhaltungen:
- DE-A1- 4 402 114
- DE-U1- 29 717 432
- US-A- 5 434 418
- US-A- 5 510 623
- US-A- 6 069 935
- US-B1- 6 404 854

## Beschreibung

Die vorliegende Erfindung betrifft einen intraoraien Sensor zur Verwendung mit einem zahnärztlichen Röntgengerät, wobei der Sensor ein in einem Gehäuse gelagertes, aus elektronischen und optischen Komponenten bestehendes Detektionselement zur Erfassung der Röntgenstrahlung aufweist.

In der zahnärztlichen Röntgendiagnostik werden in letzter Zeit vermehrt digitale Röntgensysteme eingesetzt. Gegenüber klassischen Röntgensystemen, bei denen ein strahlungsempfindlicher Röntgenfilm verwendet wird, der nach der Belichtung entwickelt werden muss, bieten die neuen digitalen Systeme den Vorteil, dass das Röntgenbild unmittelbar nach der Aufnahme auf einem Bildschirm betrachtet und ggf. näher analysiert werden kann. Röntgensysteme, die entsprechend diesen neuen digitalen Verfahren arbeiten, sind beispielsweise aus der DE 44 02 114, der DE 297 17 432 U1 oder der US 5,510,623 bekannt.

Ein üblicher digitaler Röntgensensor besteht aus einem flachen, rechteckförmigen Gehäuse, in dessen Inneren ein die Röntgenstrahlung erfassendes Detektionselement angeordnet ist. Wesentlicher Bestandteil des Detektionselements ist ein in eine Vielzahl von Pixelbereiche unterteilter Halbleiter-Chip, beispielsweise ein CCD- oder CMOS-Chip, der die Strahlung, bzw. die in sichtbares Licht umgewandelte Strahlung erfasst und hierdurch ein digitales Röntgenbild erstellt. Die von dem Halbleiter-Chip erfassten Signale werden über ein Kabel an eine Auswerteeinheit übermittelt, welche die Daten erfasst und das digitale Röntgenbild beispielsweise auf einem Bildschirm oder Display darstellt.

Üblicherweise besteht das Gehäuse eines derartigen digitalen Sensors aus zwei Kunststoffteilen, die nach Befestigung des Detektionselements in bzw. an einem der beiden Gehäuseteile zusammengefügt und miteinander verklebt werden. Dabei besteht der Nachteil, dass ein derartiger Sensor nicht sterilisiert werden kann, da das Kunststoffgehäuse und insbesondere dessen Klebeverbindungen die hohen Temperaturen einer einmaligen oder wiederholten Sterilisation nicht aushalten würden. Auch hinsichtlich der elektro-optischen Komponenten des Sensors besteht die Gefahr, dass diese beschädigt werden, wenn sie zu hohen Temperaturen ausgesetzt werden. Andererseits besteht in der Medizin allgemein ein Bedürfnis nach einem hohen hygienischen Standard. Da die Sensoren in den Mundraum eines zu untersuchenden Patienten eingebracht werden, besteht für diese Gegenstände sogar ein besonderes Bedürfnis, diese möglichst effektiv reinigen und sterilisieren zu können.

Ein weiterer Nachteil der bekannten intraoralen Sensoren besteht in der Lagerung des Detektionselements innerhalb des Gehäuses. Üblicherweise wird das Detektionselement in dem Kunststoffgehäuse entweder flächig auf einer der Bodenseiten des Gehäuses oder an den Ecken gelagert. Das Problem besteht dabei darin, dass die Detektionselemente an ihren Ecken zur Delamination, also einem Ablösen der Scintillatorschicht von dem Substrat neigen. Die Scintillatorschicht ist für die Umwandlung der Röntgenstrahlung in für den Halbleiter-Chip erfassbares sichtbares Licht notwendig. Ein Ablösen dieser Schicht von dem Substrat hat dementsprechend zur Folge, dass in diesem Bereich die Effektivität der Umwandlung der Röntgenstrahlung verändert, z.B. herabgesetzt oder u.U. sogar heraufgesetzt wird. Dies hat zur Folge, dass der Sensor in dem betroffenen Bereich eine veränderte Sensitivität aufweist, was ab einer bestimmten Größe eines derartigen Bereichs dazu führt, dass der Sensor insgesamt nicht mehr verwendet werden kann.

Das Problem der Delamination tritt insbesondere dann auf, wenn der Sensor durch Stöße erschüttert wird, beispielsweise dann, wenn er fallengelassen wird. Verstärkt wird dieses Problem bei den bekannten Sensoren ferner dadurch, dass das Kunststoffgehäuse nur unzureichend in der Lage ist, die von außen zugefügten Stöße oder Erschütterungen abzudämpfen.

Aus der US 5,434,418 ist ein intraoraler Sensor mit einem Detektionselement zur Erfassung von Röntgenstrahlung bekannt, das im Inneren eines Gehäuses gelagert ist.

Aus der US 6,404,854 B1 ist ein Röntgensensor bekannt, bei dem ein Detektionselement vor Erschütterung und Feuchtigkeit geschützt in einem Gehäuse angeordnet ist.

Aus der EP 0 933 650 A2 ist ein Röntgensensor bekannt, bei dem ein Detektionselement dadurch stoßgeschützt ist, dass es in einem Gehäuse von vielen gasgefüllten Behältern umgeben ist.

Aus der US 2002/0014594 A1 ist ein Röntgensensor bekannt, bei dem zur Lagerung des Detektionselements unterschiedliche elastische Lagerelemente vorgesehen sind.

Der vorliegenden Erfindung liegt dementsprechend die Aufgabe zugrunde, einen intraoralen Sensor zur Verwendung in der zahnärztlichen Diagnostik anzugeben, bei dem die Gefahr einer Beschädigung des Sensors, insbesondere einer Delamination des Detektionselements weitestgehend unterbunden wird.

Die Aufgabe wird durch einen intraoralen Sensor gemäß Anspruch 1 gelöst.

Gemäß der vorliegenden Erfindung ist das Detektionselement über mehrere Dämpfungselemente innerhalb des Gehäuses gelagert.

Insbesondere ist dabei das Detektionselement an zwei paarweise gegenüberliegenden Seiten über die im wesentlichen gesamte Länge mittels zweier Dämpfungselemente mit dem Sensorgehäuse verbunden.

Beispielsweise kann die Lagerung mittels Silikon erfolgen. Eine alternative Möglichkeit der Lagerung besteht darin, dass ein gegenüberliegendes Seitenpaar lediglich jeweils in der Mitte einen Lagerpunkt aufweist und eine zweite Fixierung über paarweise senkrecht dazu angeordnete Lagerpunkte erfolgt, vorzugsweise wiederum in der Mitte der beiden Sensorkanten.

Die dämpfende Lagerung des Detektionselements hat zur Folge, dass auf den Sensor ausgeübte externe Erschütterungen aufgefangen werden und damit die Gefahr einer Beschädigung deutlich reduziert wird. Selbst für den Fall, dass für das Sensorgehäuse sehr starre Materialien, wie beispielsweise Porzellan, Keramik, eine Emaillierung oder Metall verwendet werden, ist die Dämpfung noch ausreichend, um die beim Herabfallen des Sensors auftretenden Stöße und Erschütterungen abzumildern.

Andererseits besteht allerdings die Möglichkeit, die Gefahr von Beschädigungen beim Herabfallen des Sensors weiter dadurch zu reduzieren, dass das Gehäuse ebenfalls aus einem elastischen Material, beispielsweise wiederum aus Silikon besteht. Neben dem verbesserten Fallschutz kann durch die weiche Gestaltung des Sensorgehäuses ferner auch die Akzeptanz bei Patienten, welche ein steifes Element als sehr unkomfortabel empfinden und möglicherweise zum Brechreiz neigen, deutlich verbessert werden.

Eine dazu alternative Ausführungsform besteht darin, lediglich die Ecken und/oder Kanten des Sensorgehäuses aus einem elastischen oder viskoelastischen Material zu formen, wobei der Rest des Sensorgehäuses aus den zuvor genannten abriebfesten Materialien oder aus Kunststoff besteht. Diese Variante erlaubt es, die Vorteile eines abriebfesten Gehäuses mit denjenigen einer dämpfenden bzw. elastischen Ummantelung zu kombinieren. In dem letztgenannten Fall könnte das aus Kunststoff und dem elastischen bzw. viskoelastischen Material bestehende Gehäuse im sog. 2-Komponentenspritzgußverfahren hergestellt werden.

Gemäß einem bevorzugten Ausführungsbeispiel der vorliegenden Erfindung sind die Elemente des Sensors, also das Detektionselement und/oder das Gehäuse in einer Weise ausgestaltet, das die Ecken des Sensors der Anatomie des Mundraumes eines zu untersuchenden Patienten entsprechen. Dies wird beispielsweise dadurch erzielt, dass die Ecken abgerundet bzw. gekrümmt sind oder aus mehreren aneinandergesetzten abgewinkelten Bereichen bestehen.

Üblicherweise ist zumindest das Detektionselement - hiervon insbesondere der CCD-Chip oder der CMOS-Chip - rechtwinklig ausgestaltet, was sich aus der Bearbeitungstechnik durch das Sägen der Wafer für die Halbleiterchips ergibt. Um die Form des Detektionselements der Anatomie des Mundraumes anzupassen, wird der Wafer gemäß dem vorteilhaften Ausführungsbeispiel in mehreren Stufen geschnitten, wobei das Sägeblatt einer gekrümmten oder polygonen Form entsprechend geführt wird und hierdurch eine im wesentlichen abgerundete Ecke geschaffen wird. Darüber hinaus könnte der Wafer auch durch Fräsen entsprechend abgerundet werden. Die abgerundete oder polygone Form hat darüber hinaus auch den Vorteil, dass im Rahmen der anatomischen Gegebenheiten des Mundraumes für eine gegebene Detektionsfläche möglichst viele Bildinformationen erfasst werden können.

Nachfolgend soll die Erfindung anhand der beiliegenden Zeichnung näher erläutert werden. Es zeigen.
- Fig. 1: einen intraoralen Röntgensensor in perspektivischer Darstellung;
- Fig. 2: den in Fig. 1 dargestellten Sensor in Draufsicht;
- Fig. 3: einen erfindungsgemäßen Sensor im Querschnitt;
- Fig. 4a und 4b: Darstellungen gemäß dem Schnitt A-A in Fig. 3 zur Verdeutlichung der Lagermöglichkeiten des Detektionselements innerhalb des Gehäuses.

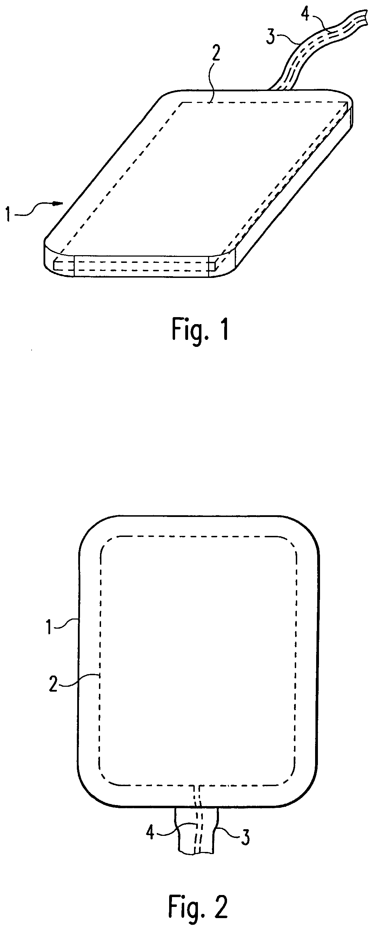

Der in den Fig. 1 und 2 dargestellte intraorale Sensor besteht im wesentlichen aus einem flachen, rechteckigen Gehäuse 1, in dessen Inneren ein Detektionselement 2 angeordnet ist. An einer Stirnseite des Gehäuses 1 ist ein Kabel 3 angeschlossen, in dem elektrische Leitungen 4 verlaufen, die an das Detektionselement 2 angeschlossen sind. Das sensorferne Ende des Kabels 3 führt zu einer - nicht dargestellten - Auswerteeinheit, welche die von den Detektionselement 2 erfassten Signale empfängt und hieraus ein digitales Bild erstellt, welches beispielsweise auf einem Display eines zahnärztliches Behandlungsplatzes dargestellt wird.

Dabei besteht das Gehäuse 1 aus einem abriebfesten Material, beispielsweise aus Porzellan, Keramik, einer Emaillierung oder Metall. Die Verwendung dieser Materialien hat zum Vorteil, dass hierdurch das Sensorgehäuse 1 gegenüber hohen Temperaturen weitestgehend unempfindlich ist und somit die Möglichkeit besteht, den Sensor zu sterilisieren. Allgemein ist das Gehäuse 1 bei Verwendung der oben genannten Materialien oder einer Mischung davon deutlich besser zu reinigen als Kunststoff und vermittelt darüber hinaus auch noch eine höhere Wertigkeit. Die bevorzugten Materialien weisen darüber hinaus auch den Vorteil auf, dass sie gasdicht sind, so dass die Möglichkeit besteht, den Sensor zusätzlich mit Ozon, welches anderenfalls die elektronischen Komponenten im Inneren des Gehäuses 1 angreifen würde, zu reinigen.

Eine Besonderheit des in den Fig. 1 und 2 dargestellten Gehäuses 1 sowie des darin angeordneten Detektionselements 2 besteht ferner darin, dass beide Elemente jeweils abgerundete Ecken aufweisen. Hierdurch wird die Form des Sensors der Anatomie des Mundraumes angepasst wodurch das Einbringen und Lagern des Sensors innerhalb des Mundraumes während der Röntgenuntersuchung für den Patienten angenehmer ist.

Die abgerundete Ausgestaltung des Sensorgehäuses 1 kann dabei ohne weitere Schwierigkeiten erfolgen. Problematischer ist hingegen, auch das Detektionselement 2, insbesondere den darauf angeordneten CCD- oder CMOS-Chip abzurunden. Die Formgebung des Chips erfolgt in der Regel durch das Sägen eines Wafers, weshalb üblicherweise rechteckige Chips mit Ecken im 90°-Winkel zum Einsatz kommen. Um auch das Detektionselement 2 abgerundet oder zumindest nahezu abgerundet auszugestalten, wird im vorliegenden Fall der Wafer in mehreren Stufen geschnitten, wobei das Sägeblatt der gewünschten gekrümmten Form entsprechend geführt wird. Alternativ dazu könnte der zunächst vollständig rechteckig geschnittene Chip anschließend auch in die abgerundete Form gefräst werden. Dabei wirkt sich zwar die abgerundete Form des Detektionselements 2 für sich alleine genommen nicht auf das Wohlbefinden des Patienten aus, diese Form ist allerdings dennoch wünschenswert, da bei der Verwendung eines abgerundeten Gehäuses 1 hierdurch der zur Verfügung stehende Bereich optimal ausgenützt wird und somit ein Höchstmaß an Bildinformationen erfaßt werden kann.

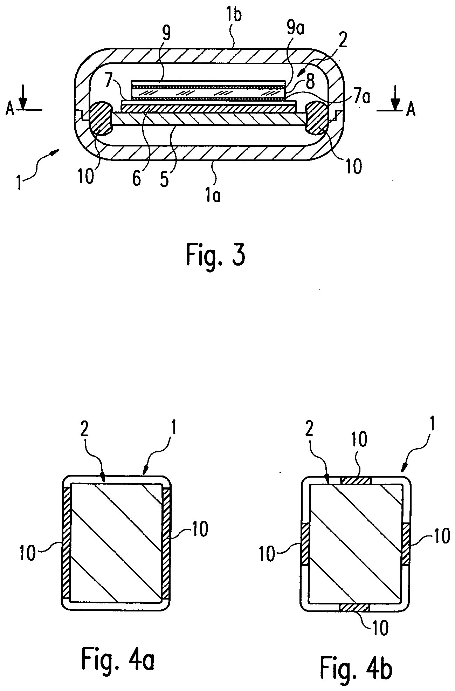

Die Verwendung der oben genannten bevorzugten Materialien Porzellan, Keramik, Emaillierung oder Metall hat zur Folge, dass hierdurch das Sensorgehäuse 1 nochmals steifer wird als bei der bisher bevorzugten Verwendung von Kunststoff. Von außen zugeführte Stöße oder Erschütterungen würden hierbei besonders effektiv auf das im Inneren des Gehäuses 1 gelagerte Detektionselement 2 übertragen werden, weshalb die erhöhte Gefahr einer Beschädigung des Detektionselements 2 besteht. Anhand der Fig. 3 und 4 sollen deshalb nachfolgend Möglichkeiten beschrieben werden, das Detektionselement 2 verbessert gegen externe Stöße und Erschütterungen zu schützen.

Fig. 3 zeigt den erfindungsgemäßen Sensor im Querschnitt. Das Detektionselement 2 besteht dabei aus einem als Trägerelement dienenden Substrat 5, auf dessen Oberseite zunächst eine die Röntgenstrahlung abschirmende Schicht 6 aufgebracht ist, wobei auf diese Abschirmschicht 6 allerdings auch verzichtet werden könnte. Auf der Oberseite dieser Abschirmschicht 6 bzw. auf der Oberseite des Substrats 5 befindet sich das lichtempfindliche Element 7, also ein CCD- oder ein CMOS-Chip zur ortsaufgelösten Erfassung von Bildinformationen.

Da der CCD-Chip oder der CMOS-Chip für sichtbares Licht deutlich empfindlicher ist als für die Röntgenstrahlung, wird die Empfindlichkeit des Detektionselements 2 dadurch erhöht, dass vor der Halbleiterschicht 7 noch eine Lichtleiterschicht 8 und eine Scintillatorschicht 9 angeordnet sind. Die Scintillatorschicht 9 dient dazu, auftreffende Röntgenstrahlung über Scintillationseffekte in sichtbares Licht umzuwandeln, welches über die Lichtleiterschicht 8 auf die Halbleiterschicht 7 übertragen wird. Die Lichtleiterschicht 8 ist dabei derart ausgestaltet, dass die an einer bestimmten Stelle der Scintillatorschicht 9 auftreffende Röntgenstrahlung auch im wesentlichen an der entsprechenden unterhalb gelegenen Stelle der Halbleiterschicht 7 als sichtbares Licht erfasst wird. Hierdurch wird somit die Detektionsempfindlichkeit des Detektionselements 2 deutlich erhöht, wobei das Auflösungsvermögen nur unwesentlich beeinträchtigt wird.

Der in Fig. 3 dargestellte Aufbau entspricht im wesentlichen dem klassischen Aufbau eines Detektionselements für einen intraoralen digitalen Sensor, wobei - wie bereits erwähnt - ggf. die Abschirmschicht 6 und/oder die Lichtleiterschicht 8 auch entfallen können. Üblicherweise ist dabei das Detektionselement in dem Sensorgehäuse an den Ecken gelagert oder flächig an einer der beiden Bodenseiten des Gehäuseunterteils 1a oder des Gehäuseoberteils 1b befestigt. Bei dieser üblicherweise verwendeten Lagerung werden allerdings auf das Gehäuse ausgeübte externe Stöße oder Erschütterungen nahezu unvermindert auf das Detektionselement übertragen, wobei die Gefahr besteht, dass das Detektionselement beschädigt wird, insbesondere, dass sich die Scintillatorschicht 9 ablöst.

In diesem Zusammenhang ist zu berücksichtigen, dass die Halbleiterschicht 7 mit der Lichtleiterschicht 8 bzw. die Lichtleiterschicht 8 mit der Scintillatorschicht 9 über sich jeweils dazwischen befindende Klebe- bzw. Kopplungsschichten 7a und 9a zusammengehalten werden. Für den Fall, dass auf die Lichtleiterschicht 8 verzichtet wird, wird die Scintillatorschicht 9 üblicherweise auf einem eigenen (nicht dargestellten) Substrat hergestellt, wobei dieser Verbund bestehen aus der Scintillatorschicht 9 und dem zugehörigen Substrat wiederum mittels der Klebeschicht 7a auf die Halbleiterschicht 7 aufgeklebt wird. Wird nun diese Anordnung Erschütterungen _{O}uci starken Stößen ausgesetzt, so besteht mc Gefahr, dass mc Kopplungswirkung der Klebe- bzw. Kopplungsschichten 7a und 9a nachläßt und sich die verschiedenen Schichten des Verbandes voneinander lösen. Hierdurch kann die Sensitivität des Sensors partiell verändert werden, so dass der Sensor schließlich unbrauchbar wird.

Anzumerken ist ferner, dass anstelle der Verwendung von Klebeschichten auch die Möglichkeit besteht, die Scintillatorschicht 9 auf die Lichtleiterschicht 8 bzw. die Halbleiterschicht 7 aufzudrucken. Auch bei dieser Variante besteht allerdings die Gefahr, dass sich die Scintillatorschicht 9 aufgrund von Erschütterungen von ihrer Unterlage löst und damit der Sensor beschädigt wird.

Um daher auch bei den zuvor erwähnten besonders starren Materialien für das Gehäuse 1 eine Beschädigung des Detektionselements 2 zu verhindern, ist dieses über eine besondere Dämpfung innerhalb des Gehäuses 1 gelagert. Die besondere Lagerung wird durch zwei oder vier Dämpfungselemente 10 erreicht, über welche die Seitenkanten des Detektionselements 2 mit den entsprechenden Innenwänden des Sensorgehäuses 1 verbunden sind. Die Dämpfungselemente 10 bestehen aus einem elastischen oder viskoelastischen Material mit einer inhärenten Dämpfung, beispielsweise aus Silikon.

Bei einer ersten Variante der erfindungsgemäßen Lagerung, die in Fig. 4a schematisch dargestellt ist, sind zwei einander gegenüberliegende Seiten des Detektionselements 2 über ihre gesamte Länge hinweg über zwei Dämpfungselemente 10 mit dem Gehäuse 1 verbunden. Eine hierzu alternative, in Fig. 4b dargestellte zweite erfindungsgemäße Lagerung besteht darin, zwei einander gegenüberliegende Seiten jeweils nur in ihrem mittleren Bereich über zwei kurze Dämpfungselemente 10 mit dem Sensorgehäuse 1 zu verbinden und darüber hinaus an den weiteren, senkrecht dazu stehenden Seiten ebenfalls zwei Dämpfungselemente 10 anzuordnen. Diese sind wiederum vorzugsweise jeweils in der Mitte der Kanten des Detektionselements 2 angeordnet.

Die erfindungsgemäße dämpfende Lagerung des Detektionselements 2 innerhalb des Sensorgehäuses 1 hat zur Folge, dass auf das Gehäuse ausgeübte externe Erschütterungen abgefangen werden und somit die Gefahr einer Beschädigung des Detektionselements 2 weitestgehend unterbunden wird. Dies ist insbesondere dann von Bedeutung, wenn das Sensorgehäuse aus einem selbst nicht dämpfenden Material, beispielsweise aus Porzellan, Keramik oder Metall besteht. Die Verwendung eines der zuvor genannten Materialien sowie der erfindungsgemäßen Dämpfungslagerung ermöglicht somit die Realisierung eines intraoralen Sensors, der einerseits besonders gut und effektiv zu reinigen ist und bei dem andererseits die Gefahr einer Beschädigung des Detektionselements durch externe Stöße oder Erschütterungen vermieden wird.

Wird hingehen primär angestrebt, eine Beschädigung des Detektionselements bei einem Herabfallen des Sensors zu vermeiden, so können zusätzlich auch das Gehäuse bzw. dessen Ecken und/oder Kanten aus einem weichen oder dämpfenden Material, beispielsweise aus Silikon bestehen. Die Verwendung eines entsprechenden Materials auch für das Sensorgehäuse hätte darüber hinaus auch den Vorteil, dass dieses für Patienten, insbesondere für ältere Patienten oder Kinder deutlich angenehmer ist und somit die Akzeptanz der digitalen Röntgentechnik in der zahnärztlichen Diagnostik erhöht werden kann.

## Patentansprüche

1. Intraoraler Sensor für ein zahnärztliches Röntgengerät, aufweisend ein Detektionselement (2) zur Erfassung der Röntgenstrahlung, das im Inneren eines Gehäuses (1) gelagert ist,
wobei das Detektionselement (2) über Dämpfungselemente (10) innerhalb des Gehäuses (1) gelagert ist, welche aus einem elastischen oder viskoelastischen Material bestehen,
und wobei das Gehäuse eine flache, rechteckige Form aufweist und das Detektionselement (2)
an zwei einander gegenüberliegenden Seitenflächen über die im wesentlichen gesamte Länge hinweg mittels zweier Dämpfungselemente (10) mit den entsprechenden Seitenwänden des Gehäuses verbunden ist oder
über vier Dämpfungselemente (10) mit dem Gehäuse (1) verbunden ist, wobei zwei Dämpfungselemente (10) an zwei einander gegenüberliegenden Seitenflächen und die beiden weiteren Dämpfungselemente (10) an zwei senkrecht dazu angeordneten Seitenflächen des Detektionselements (2) angeordnet sind.

2. Intraoraler Sensor nach Anspruch 1,
**dadurch gekennzeichnet,**
**dass** das Gehäuse (1) bzw. Ecken und/oder Kanten davon aus einem elastischen oder viskoelastischen Material besteht.

3. Intraoraler Sensor nach Anspruch 2,
**dadurch gekennzeichnet,**
**dass** das Gehäuse (1) bzw. dessen elastischen Ecken und/oder Kanten aus Silikon besteht.

4. Intraoraler Sensor nach einem der Ansprüche 1 bis 3,
**dadurch gekennzeichnet,**
**dass** die Dämpfungselemente (10) aus Silikon bestehen.

5. Intraoraler Sensor nach einem der vorherigen Ansprüche,
**dadurch gekennzeichnet,**
**dass** vier Dämpfungselemente (10) jeweils in der Mitte der zugeordneten Seitenfläche angeordnet sind.

6. Intraoraler Sensor nach einem der vorherigen Ansprüche,
**dadurch gekennzeichnet,**
**dass** das Detektionselement (2) und/oder das Gehäuse (1) entsprechend der Anatomie des Mundraumes gestaltet sind.

7. Intraoraler Sensor nach Anspruch 6,
**dadurch gekennzeichnet,**
**dass** die Ecken des Detektionselement (2) und/oder des Gehäuses (1) abgerundet sind oder eine polygone Form aufweisen.

8. Intraoraler Sensor nach Anspruch 6,
**dadurch gekennzeichnet,**
**dass** das Detektionselement (2) ein lichtempfindliches Halbleiterelement (7) mit einer rechteckigen Grundform aufweist, dessen Ecken abgerundet sind bzw. aus mehreren aneinandergesetzten abgewinkelten Bereichen bestehen.

9. Intraoraler Sensor nach einem der vorhergehenden Ansprüche,
**dadurch gekennzeichnet,**
**dass** das Detektionselement (2) ein lichtempfindliches Halbleiterelement (7) aufweist, bei dem es sich um einen CCD- oder einen CMOS-Chip handelt.

10. Intraoraler Sensor nach Anspruch 9,
**dadurch gekennzeichnet,**
**dass** vor dem Halbleiterelement (7) eine Scintillatorschicht (9) angeordnet ist.

11. Intraoraler Sensor nach Anspruch 10,
**dadurch gekennzeichnet,**
**dass** zwischen dem Halbleiterelement (7) und der Scintillatorschicht (9) eine Lichtleiterschicht (8) angeordnet ist.

12. Intraoraler Sensor nach einem der vorhergehenden Ansprüche,
**dadurch gekennzeichnet,**
**dass** das Gehäuse (1) gasdicht ist.

## Claims

1. Intraoral sensor for a dental X-ray apparatus, having a detection element (2) for detecting the X-ray radiation, which detection element (2) is mounted in the interior of a housing (1), wherein the detection element (2) is mounted inside the housing (1) via damping elements (10), which are made of an elastic or viscoelastic material, and wherein the housing has a flat rectangular shape, and the detection element (2), on two mutually opposite side surfaces, is connected to the corresponding side walls of the housing substantially along the entire length by means of two damping elements (10), or is connected to the housing (1) via four damping elements (10), wherein two damping elements (10) are arranged on two mutually opposite side surfaces, and the two further damping elements (10) are arranged on two side surfaces of the detection element (2) that are arranged perpendicularly thereto.

2. Intraoral sensor according to Claim 1, **characterized in that** the housing (1) or corners and/or edges thereof is/are made of an elastic or viscoelastic material.

3. Intraoral sensor according to Claim 2, **characterized in that** the housing (1) or the elastic corners and/or edges thereof is/are made of silicone.

4. Intraoral sensor according to one of Claims 1 to 3, **characterized in that** the damping elements (10) are made of silicone.

5. Intraoral sensor according to one of the preceding claims, **characterized in that** four damping elements (10) are each arranged in the middle of the associated side surface.

6. Intraoral sensor according to one of the preceding claims, **characterized in that** the detection element (2) and/or the housing (1) are/is configured according to the anatomy of the oral cavity.

7. Intraoral sensor according to Claim 6, **characterized in that** the corners of the detection element (2) and/or of the housing (1) are rounded or have a polygonal shape.

8. Intraoral sensor according to Claim 6, **characterized in that** the detection element (2) has a light-sensitive semiconductor element (7), which has a rectangular basic shape and of which the corners are rounded or are composed of a plurality of juxtaposed angled regions.

9. Intraoral sensor according to one of the preceding claims, **characterized in that** the detection element (2) has a light-sensitive semiconductor element (7) in the form of a CCD chip or a CMOS chip.

10. Intraoral sensor according to Claim 9, **characterized in that** a scintillator layer (9) is arranged in front of the semiconductor element (7).

11. Intraoral sensor according to Claim 10, **characterized in that** an optical waveguide layer (8) is arranged between the semiconductor element (7) and the scintillator layer (9).

12. Intraoral sensor according to one of the preceding claims, **characterized in that** the housing (1) is gas-tight.

## Revendications

1. Détecteur intraoral pour un appareil à rayons X dentaire, présentant un élément de détection (2) pour la détection du rayonnement X, qui est monté à l'intérieur d'un boîtier (1),
dans lequel l'élément de détection (2) est monté à l'intérieur du boîtier (1) au moyen d'éléments d'amortissement (10), qui se composent d'un matériau élastique ou viscoélastique, et
dans lequel le boîtier présente une forme rectangulaire plate et l'élément de détection (2)
est relié sur deux faces latérales opposées l'une à l'autre sur essentiellement toute la longueur aux parois latérales correspondantes du boîtier au moyen de deux éléments d'amortissement (10) ou
est relié au boîtier (1) au moyen de quatre éléments d'amortissement (10), dans lequel deux éléments d'amortissement (10) sont disposés sur deux faces latérales opposées l'une à l'autre et les deux autres éléments d'amortissement (10) sont disposés sur deux faces latérales de l'élément de détection (2) disposées perpendiculairement à celles-ci.

2. Détecteur intraoral selon la revendication 1, **caractérisé en ce que** le boîtier (1) ou des coins et/ou des côtés de celui-ci est/sont constitué(s) d'un matériau élastique ou viscoélastique.

3. Détecteur intraoral selon la revendication 2, **caractérisé en ce que** le boîtier (1) ou ses coins et/ou côtés élastiques est/sont constitué(s) de silicone.

4. Détecteur intraoral selon l'une quelconque des revendications 1 à 3, **caractérisé en ce que** les éléments d'amortissement (10) sont constitués de silicone.

5. Détecteur intraoral selon l'une quelconque des revendications précédentes, **caractérisé en ce que** quatre éléments d'amortissement (10) sont disposés respectivement au milieu de la face latérale associée.

6. Détecteur intraoral selon l'une quelconque des revendications précédentes, **caractérisé en ce que** l'élément de détection (2) et/ou le boîtier (1) sont configurés conformément à l'anatomie de la cavité buccale.

7. Détecteur intraoral selon la revendication 6, **caractérisé en ce que** les coins de l'élément de détection (2) et/ou du boîtier (1) sont arrondis ou présentent une forme polygonale.

8. Détecteur intraoral selon la revendication 6, **caractérisé en ce que** l'élément de détection (2) présente un élément semi-conducteur sensible à la lumière (7) avec une forme de base rectangulaire, dont les coins sont arrondis ou se composent de plusieurs zones coudées reliées entre elles.

9. Détecteur intraoral selon l'une quelconque des revendications précédentes, **caractérisé en ce que** l'élément de détection (2) présente un élément semi-conducteur sensible à la lumière (7), qui est une puce CCD ou CMOS.

10. Détecteur intraoral selon la revendication 9, **caractérisé en ce qu'**une couche de scintillateur (9) est disposée devant l'élément semi-conducteur (7).

11. Détecteur intraoral selon la revendication 10, **caractérisé en ce qu'**une couche de guide d'onde lumineuse (8) est disposée entre l'élément semiconducteur (7) et la couche de scintillateur (9).

12. Détecteur intraoral selon l'une quelconque des revendications précédentes, **caractérisé en ce que** le boîtier (1) est étanche au gaz.
